# EUROPÄISCHE PATENTSCHRIFT

(11) **EP 4 228 866 B1**
(45) Veröffentlichungstag und Bekanntmachung des Hinweises auf die Patenterteilung: **28.10.2026**
(21) Anmeldenummer: 21802596.3
(22) Anmeldetag: 15.10.2021
(51) Int. Cl.: A61H 3/00, B25J 9/00, A61F 5/01, A61H 1/02

(54) **UNTERSTÜTZUNGSVORRICHTUNG**
SUPPORT APPARATUS
DISPOSITIF DE SOUTIEN

(30) Priorität: 15.10.2020 DE 102020006331
(43) Veröffentlichungstag der Anmeldung: 23.08.2023
(73) Patentinhaber: Spanset Inter AG, 8832 Wollerau (CH)
(72) Erfinder: EHNIMB, David, 8800 Thalwill (CH)
(74) Vertreter: Taubert, Diana
(86) Internationale Anmeldenummer: PCT/EP2021/078631
(87) Internationale Veröffentlichungsnummer: WO 2022/079252

(56) Entgegenhaltungen:
- EP-A1- 2 898 861
- EP-A2- 2 301 485
- DE-A1- 102009 042 713
- DE-A1- 102014 011 373
- US-A1- 2014 047 623
- US-A1- 2018 296 373
- US-A1- 2019 151 120
- US-A1- 2019 388 250

## Beschreibung

Die Erfindung betrifft eine Vorrichtung zur Unterstützung menschlicher Bewegungen, sogenannte Exoskelette, oder zur Absturzsicherung, wobei die Vorrichtung an einen Körper eines Menschen anordnenbar ist, die Vorrichtung über Kontaktflächen mit dem Körper verfügt und an den Kontaktflächen Kontaktelemente vorhanden / angeordnet sind, die ganz oder teilweise aus einem Phasenwechselmaterial, einem PCM (phase change material), bestehen. Vorrichtungen zur Unterstützung menschlicher Bewegungen (Exoskelett) oder Absturzsicherung sind seit langem bekannt. Bei den Produkten ist ein komfortabler Tragekomfort von besonderer Bedeutung. Gerade wenn solche Vorrichtungen getragen werden, kommt es zur zusätzlichen Verdeckung des Körpers des Menschen. In einem aktiven Zustand, auch als Bewegungszustand bezeichnet, kann thermische Energie vom Körper schlechter abgeführt werden. Der Mensch beginnt an zu schwitzen und empfindet die thermische Energie als unangenehm.

Grundsätzlich ist der Einsatz von PCM's bei Prothesen, so in der DE 10 2009 042 713 A1 und Orthesen und auch Exoskeletten bekannt, so aus der DE 10 2014 011 373 A1, welche den nächsten Stand der Technik darstellt.

Das Phasenwechselmaterial hat dabei die Eigenschaft, dass es bei Körpertemperatur eines Menschen schmilzt oder verdunstet. Für diesen Prozess bedarf es thermischer Energie, welche die Körpertemperatur des Menschen liefert, so wie vorbeschrieben im Bewegungszustand des Menschen. Während des Schmelzens oder Verdunstens wird dadurch eine Kühlung beim Menschen an Kontaktflächen der Vorrichtung wahrnehmbar. Dieser Effekt steht jedoch nicht unbegrenzt zur Verfügung. Die Aufgabe der Erfindung besteht darin, eine Vorrichtung zur Unterstützung menschlicher Bewegungen oder zur Absturzsicherung bereitzustellen, die gegenüber bisherigen Vorrichtungen optimiert ist.

Diese Aufgabe wird erfindungsgemäß durch eine Vorrichtung mit den Merkmalen des Hauptanspruchs 1 gelöst, wobei die Unteransprüche weitere erfindungsgemäße Ausgestaltungsvarianten dieser Lösungen beinhalten.

Danach weist eine Vorrichtung zur Absturzsicherung eines Menschen oder zur Unterstützung der menschlichen Bewegung, ein sogenanntes Exoskelett, wobei die Vorrichtung an einen Körper des Menschen anordnenbar ist, folgende Merkmale auf:
Die Vorrichtung verfügt über Kontaktflächen mit dem Körper. Die Kontaktflächen werden ganz oder teilweise durch mindestens ein Kontaktelement gebildet. Das mindestens eine Kontaktelement besteht ganz oder teilweise aus einem PCM (phase change material), wobei das mindestens eine Kontaktelement im Maß einer Auflage an dem Körper des Menschen veränderbar ist. Insofern ist die Auflagefläche des Kontaktelements veränderbar. Zu beachten ist, dass Kontaktflächen im Sinne der Erfindung auch nur eine Kontaktfläche sein kann. Die Formulierung wurde lediglich der Übersichtlichkeit innerhalb dieser Beschreibung so gewählt.

Erfindungsgemäß weist das mindestens eine Kontaktelement im Ruhezustand der Vorrichtung oder des Menschen an der dem Körper des Menschen zugewandten Seite eine geringere Auflagefläche und die Vorrichtung damit eine geringere Kontaktfläche auf als im Bewegungszustand. Dadurch kommt es zu einem verzögerten Phasenwechsel des PCM im Ruhezustand. Die Vorrichtung liegt dann beispielhaft weniger straff am Körper an oder Kontaktelemente haben eine Form, welche eine geringe Auflagefläche gewährt, zum Beispiel Gegenform zur Körperform oder Körperteilform.

Dadurch ist es möglich, den Phasenwechsel der PCM's zeitlich zu verlängern beziehungsweise sogar bedingt zu steuern, sodass eine Kühlung bei Einsatz der Vorrichtung optimiert werden kann.

Ferner kann vorgesehen werden, dass das mindestens eine Kontaktelement in seinem Maß der Auflage an dem Körper in Abhängigkeit von einem Ruhe- und einem Bewegungszustand des Menschen oder der Vorrichtung zur Unterstützung menschlicher Bewegung, also dem Exoskelett, veränderbar ist. Speziell bei Absturzsicherungen kommt es auf den Bewegungszustand des Menschen an und bei Exoskeletten kann es auch zusätzlich auf den Zustand der Vorrichtung selbst ankommen, also ob diese aktiviert ist oder nicht.

Es kann auch vorgesehen sein, dass mindestens ein Kontaktelement an der dem Körper des Menschen zugewandten Seite im Ruhezustand der Vorrichtung oder des Menschen Erhebungen und Vertiefungen aufweist, unebene Fläche. Dies hat den Vorteil, dass es auch hier zu einem verzögerten Phasenwechsel im Ruhezustand kommt, weil die Auflagefläche im Ruhezustand gegenüber dem Bewegungszustand vergrößert ist. Im Bewegungszustand besitzt sodann die Vorrichtung an der dem Körper des Menschen zugewandten Seite keine oder geringere Erhebungen und Vertiefungen. Hier besteht sodann der Vorteil einer verstärkten Kühlung im Bewegungszustand.

Des Weiteren kann vorgesehen sein, dass die Regulierung des Maßes der Auflage des mindestens einen Kontaktelements an dem Körper mittels Druckbeaufschlagung des mindestens einen Kontaktelements erfolgt. So kann eine Regulierung der Erhebungen und Vertiefungen oder des Maßes der Auflage des mindestens einen Kontaktelements an dem Körper des Menschen erfolgen. Die Druckbeaufschlagung kann durch jegliche geeignete und dem Fachmann bekannte Mittel erfolgen.

Insofern kann die Regulierung des Maßes der Auflage des mindestens einen Kontaktelements an dem Körper und damit auch die Druckbeaufschlagung des mindestens einen Kontaktelements pneumatisch, vorzugsweise mittels mindestens einer Volumen regulierbaren Luftkammer erfolgen. Durch eine Verformung der mindestens einen Luftkammer infolge einer pneumatischen Druckbeaufschlagung wird ebenfalls auf das mindestens eine Kontaktelement eingewirkt, bei dem sich so das Maß der Auflage oder auch die Ebenheit der dem Körper des Menschen zugewandten Seite des Kontaktelements verändert.

Das mindestens eine Kontaktelement steht insofern mit der mindestens einen Luftkammer in Wirkverbindung. Das mindestens eine Kontaktelement ist zum Beispiel mit der mindestens einen Luftkammer unterfüttert oder Kontaktelemente wechseln sich mit der mindestens einen Luftkammer ab. Es ist so möglich, die Kontaktfläche der Kontaktelemente über die Ausdehnung der Luftkammern auf einfache Art zu steuern. Die konkrete Anordnung der mindestens einen Luftkammer als solche ist aber nicht vorgegeben. Dies hängt von dem jeweiligen Aufbau der Vorrichtung ab.

Ferner kann die Regulierung des Volumens der mindestens einen Luftkammer, also die pneumatische Druckbeaufschlagung, mittels einer Pumpe, beispielhaft einer Handpumpe oder einer maschinellen Pumpe und einer Ventilsteuerung, erfolgen. Maschinelle Pumpen sind alle bekannten Pumpen, die nicht handbetrieben sind. Die Pumpen können an der Vorrichtung unmittelbar angebracht sein oder es können externe Pumpen zum Einsatz kommen. Die Ventilsteuerung kann beispielhaft an der Pumpe selbst oder an der mindestens einen Luftkammer oder an Leitungen zwischen Luftkammer und Pumpe angeordnet sein, so zum Beispiel als Ablassventil, um eine pneumatische Druckbeaufschlagung mittels der Pumpe wieder zu verringern. Ventilsteuerung im Sinne der Erfindung sind alle pneumatischen Steuerungen, die der Fachmann in konkreten Fall als geeignet ansieht, um eine Druckregulierung neben der Pumpe zu gewähren.

Eine weitere Möglichkeit der Regulierung des Volumens der mindestens einen Luftkammer besteht darin, dass eine Druckbeaufschlagung der mindestens einen Luftkammer durch mindestens eine weitere Luftkammer erfolgt, die mit der mindestens einen Luftkammer verbunden ist, wobei die mindestens eine weitere Luftkammer durch eine Bewegung des Menschen bei der Benutzung der Vorrichtung ihr Volumen verringert und so die mindestens eine Luftkammer druckbeaufschlagt wird. Es handelt sich somit um korrespondierende Luftkammern, die je nach Bewegung des Menschen und damit auch der Vorrichtung ihr Volumen verändern.

Ebenfalls kommt in Betracht, dass das mindestens eine Kontaktelement Teil einer Abstütz- und/oder Ablagevorrichtung der Vorrichtung für Körperteile des Menschen ist, so beispielhaft einer Kopfstütze. Wenn an einer Kopfstütze mindestens ein Kontaktelement angebracht ist, dann kann der die Vorrichtung nutzende Mensch bei einer Ruhephase den Kopf ablegen und kühlen.

Wie bereits dargestellt, ist es durch die vorbeschriebene Vorrichtung möglich, den Phasenwechsel der PCM's zeitlich zu verlängern beziehungsweise sogar bedingt zu steuern, sodass eine Kühlung bei Einsatz der Vorrichtung optimiert werden kann.

Die Erfindung wird nachfolgend anhand eines Ausführungsbeispiels unter Bezugnahme auf die Figuren weiter erläutert. Dabei ergeben sich weitere Vorteile, Merkmale und Ausgestaltungen der Erfindung.

Es zeigen:
**Fig. 1** eine schematische Darstellung einer Vorrichtung im Ruhezustand,
**Fig. 2** eine schematische Darstellung einer Vorrichtung im Bewegungszustand.

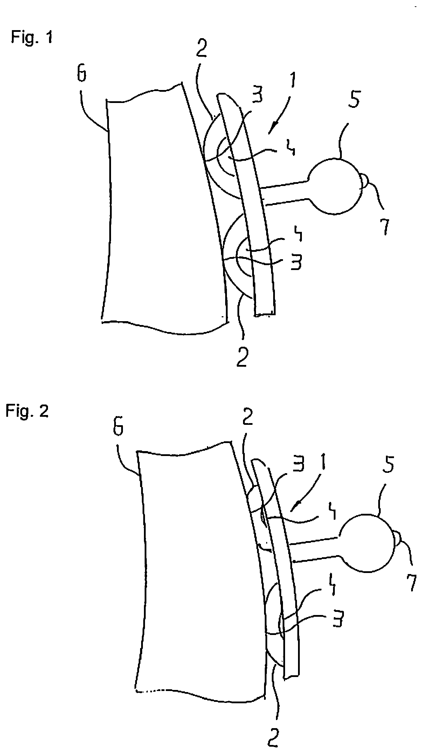

In **Fig. 1** und **Fig. 2** ist ein Teil einer erfindungsgemäßen Vorrichtung **1** in Form eines Exoskeletts dargestellt. Die Vorrichtung **1** ist an einen Körper eines Menschen **6** angeordnet und weist Kontaktflächen **3** mit dem Körper des Menschen **6** auf. Die Kontaktflächen werden durch zwei Kontaktelemente **2** gebildet, die aus einem PCM (phase change material) bestehen beziehungsweise dieses beinhalten. Es liegt danach ein bekannter Aufbau eines Kontaktelements aus PCM in Form eines Kissens vor.

Die Regulierung des Maßes der Auflage der beiden Kontaktelemente **2** an dem Körper erfolgt im Ausführungsbeispiel mittels Druckbeaufschlagung der Kontaktelemente **2.** Dies wird im Ausführungsbeispiel pneumatisch mittels zweier Volumen regulierbarer Luftkammern **4** gewährleistet, welche die beiden Kontaktelemente **2** jeweils unterfüttern. So steht die jeweilige Luftkammer **4** mit dem jeweiligen Kontaktelement **2** in einer Wirkverbindung. Die Regulierung des Volumens der beiden Luftkammern **4** und damit die pneumatische Druckbeaufschlagung erfolgt im Ausführungsbeispiel mittels einer Pumpe **5** mit Ventilsteuerung **7,** im Ausführungsbeispiel einer Handpumpe, die an der Vorrichtung **1** angeordnet und leicht zugänglich ist. Die Ventilsteuerung **7** ist im Ausführungsbeispiel ein Ablassventil, das über einen Druckknopf aktivierbar ist.

Je nach pneumatischer Druckbeaufschlagung der beiden Luftkammern **4** mittels der Pumpe **5** und der Ventilsteuerung **7** ist das Maß der Auflage der beiden Kontaktelemente **2** an den Körper des Menschen **6** veränderbar. Insofern ist die Auflagefläche des Kontaktelements veränderbar und damit die Kontaktfläche **2** der Vorrichtung **1.**

So ist gewährbar, dass die beiden Kontaktelemente an der dem Körper des Menschen **6** zugewandten Seite im Ruhezustand, **Fig. 1** eine geringere Auflagefläche aufweisen als im Bewegungszustand, **Fig. 2****.** Dadurch ist es möglich, den Phasenwechsel des in den Kontaktelementen enthaltenen Phasenwechselmaterials (PCM) zeitlich zu verlängern und mittels der Handpumpe mit Ventilsteuerung bedingt zu steuern, sodass eine Kühlung bei Einsatz der Vorrichtung optimiert werden kann.

### Bezugszeichenliste:

1. Vorrichtung
2. Kontaktelemente
3. Kontaktfläche
4. Luftkammer
5. Pumpe
6. Mensch (Ausschnitt)
7. Ventilsteuerung

## Patentansprüche

1. Vorrichtung (1) zur Unterstützung menschlicher Bewegungen oder zur Absturzsicherung, wobei die Vorrichtung (1) an einen Körper eines Menschen (6) anordnenbar ist, die Vorrichtung (1) über Kontaktflächen (3) mit dem Körper verfügt und die Kontaktflächen (3) ganz oder teilweise durch mindestens ein Kontaktelement (2) gebildet werden, das mindestens eine Kontaktelement (2) ganz oder teilweise aus einem PCM besteht, **dadurch gekennzeichnet, dass** das mindestens eine Kontaktelement (2) im Maß einer Auflage an dem Körper veränderbar ist, und wobei das mindestens eine Kontaktelement (2) an der dem Körper des Menschen (6) zugewandten Seite im Ruhezustand eine geringere Auflagefläche aufweist als im Bewegungszustand, wodurch es zu einem verzögerten Phasenwechsel des PCM im Ruhezustand kommt.

2. Vorrichtung (1) nach Anspruch 1, wobei das mindestens eine Kontaktelement (2) in seinem Maß der Auflage an dem Körper in Abhängigkeit von einem Ruhe- und einem Bewegungszustand des Menschen (6) oder der Vorrichtung (1) zur Unterstützung menschlicher Bewegung veränderbar ist.

3. Vorrichtung (1) nach Anspruch 1 oder 2, wobei das mindestens eine Kontaktelement (2) an der dem Körper des Menschen (6) zugewandten Seite im Ruhezustand Erhebungen und Vertiefungen aufweist und im Bewegungszustand an der dem Körper des Menschen (6) zugewandten Seite keine oder geringere Erhebungen und Vertiefungen besitzt.

4. Vorrichtung (1) nach einem der vorherigen Ansprüche, wobei die Regulierung des Maßes der Auflage des mindestens einen Kontaktelements (2) an dem Körper mittels Druckbeaufschlagung des mindestens einen Kontaktelements (2) erfolgt.

5. Vorrichtung (1) nach einem der vorherigen Ansprüche, wobei die Regulierung des Maßes der Auflage des mindestens einen Kontaktelements (2) an dem Körper pneumatisch, vorzugsweise mittels mindestens einer Volumen regulierbaren Luftkammer (4) erfolgt.

6. Vorrichtung (1) nach Anspruch 5, wobei das mindestens eine Kontaktelement (2) mit der mindestens einen Luftkammer (4) in Wirkverbindung steht.

7. Vorrichtung (1) nach einem der Ansprüche 5 oder 6, wobei die Regulierung des Volumens der mindestens einen Luftkammer (4), mittels einer Pumpe (5) und einer Ventilsteuerung (7) erfolgt.

8. Vorrichtung (1) nach einem der Ansprüche 5 bis 7, wobei eine Druckbeaufschlagung der mindestens einen Luftkammer (4) durch mindestens eine weitere Luftkammer erfolgt, die mit der mindestens einen Luftkammer (4) verbunden ist, wobei die mindestens eine weitere Luftkammer durch eine Bewegung des Menschen (6) bei der Benutzung der Vorrichtung (1) ihr Volumen verringert und so die mindestens eine Luftkammer (4) druckbeaufschlagt wird.

9. Vorrichtung (1) nach einem der vorherigen Ansprüche, wobei das mindestens eine Kontaktelement (2) Teil einer Abstütz- und/oder Ablagevorrichtung der Vorrichtung (1) für Körperteile des Menschen (6) ist.

## Claims

1. An apparatus (1) for supporting human movements or for protecting against falls, wherein the apparatus (1) can be arranged on the body of a person (6), the apparatus (1) has contact surfaces (3) with the body, and the contact surfaces (3) are formed completely or partially by at least one contact element (2), the at least one contact element (2) consists completely or partially of a PCM, **characterized in that** the extent to which the at least one contact element (2) bears on the body can be modified, and wherein the at least one contact element (2) on the side facing the body of the person (6) has a smaller bearing surface in the resting state than in the movement state, which results in a delayed phase change of the PCM in the resting state.

2. The apparatus (1) according to Claim 1, wherein the at least one contact element (2) can be modified in the extent to which it bears on the body as a function of a resting and a movement state of the person (6) or of the apparatus (1) for supporting human movement.

3. The apparatus (1) according to Claim 1 or 2, wherein the at least one contact element (2) has elevations and depressions on the side facing the body of the person (6) in the resting state and has no or fewer elevations and depressions on the side facing the body of the person (6) in the movement state.

4. The apparatus (1) according to any one of the preceding claims, wherein the extent to which the at least one contact element (2) bears on the body is regulated by means of pressurization of the at least one contact element (2).

5. The apparatus (1) according to any one of the preceding claims, wherein the extent to which the least one contact element (2) bears on the body is regulated pneumatically, preferably by means of at least one volume-regulatable air chamber (4).

6. The apparatus (1) according to Claim 5, wherein the at least one contact element (2) is in operative connection with the at least one air chamber (4).

7. The apparatus (1) according to any one of Claims 5 or 6, wherein the volume of the at least one air chamber (4) is regulated by means of a pump (5) and a valve control (**7**).

8. The apparatus (1) according to any one of Claims 5 to 7, wherein a pressurization of the at least one air chamber (4) is carried out by at least one further air chamber which is connected to the at least one air chamber (4), wherein the at least one further air chamber is reduced in volume by a movement of the person (6) when utilizing the apparatus (1) and thus the at least one air chamber (4) is pressurized.

9. The apparatus (1) according to any one of the preceding claims, wherein the at least one contact element (2) is part of a support and/or application apparatus of the apparatus (1) for body parts of the person (6).

## Revendications

1. Dispositif (1) destiné à soutenir des mouvements humains ou à assurer une protection contre les chutes, le dispositif (1) pouvant être disposé sur le corps d'un être humain (6), le dispositif (1) comportant des surfaces de contact (3) avec le corps et les surfaces de contact (3) étant formées entièrement ou partiellement par au moins un élément de contact (2), l'au moins un élément de contact (2) étant constitué entièrement ou partiellement d'un PCM, **caractérisé en ce que** l'au moins un élément de contact (2) est modifiable dans le degré d'un appui sur le corps, et l'au moins un élément de contact (2) présentant, sur le côté tourné vers le corps de l'être humain (6), une surface d'appui plus faible à l'état de repos qu'à l'état de mouvement, ce qui entraîne un changement de phase retardé du PCM à l'état de repos.

2. Dispositif (1) selon la revendication 1, l'au moins un élément de contact (2) étant modifiable dans son degré de l'appui sur le corps en fonction d'un état de repos et d'un état de mouvement de l'être humain (6) ou du dispositif (1) destiné à soutenir un mouvement humain.

3. Dispositif (1) selon la revendication 1 ou 2, l'au moins un élément de contact (2) présentant, sur le côté tourné vers le corps de l'être humain (6), des reliefs et des creux à l'état de repos et ne présentant pas de reliefs et de creux, ou présentant des reliefs et des creux plus faibles, sur le côté tourné vers le corps de l'être humain (6), à l'état de mouvement.

4. Dispositif (1) selon l'une quelconque des revendications précédentes, la régulation du degré de l'appui de l'au moins un élément de contact (2) sur le corps étant effectuée au moyen d'une mise sous pression de l'au moins un élément de contact (2).

5. Dispositif (1) selon l'une quelconque des revendications précédentes, la régulation du degré de l'appui de l'au moins un élément de contact (2) sur le corps étant effectuée de manière pneumatique, de préférence au moyen d'au moins une chambre à air (4) à volume réglable.

6. Dispositif (1) selon la revendication 5, l'au moins un élément de contact (2) étant en liaison fonctionnelle avec l'au moins une chambre à air (4).

7. Dispositif (1) selon l'une quelconque des revendications 5 ou 6, la régulation du volume de l'au moins une chambre à air (4) étant effectuée au moyen d'une pompe (5) et d'une commande de soupape (7).

8. Dispositif (1) selon l'une quelconque des revendications 5 à 7, une mise sous pression de l'au moins une chambre à air (4) étant effectuée par au moins une autre chambre à air reliée à l'au moins une chambre à air (4), l'au moins une autre chambre à air réduisant son volume par un mouvement de l'être humain (6) lors de l'utilisation du dispositif (1), et l'au moins une chambre à air (4) étant ainsi mise sous pression.

9. Dispositif (1) selon l'une quelconque des revendications précédentes, l'au moins un élément de contact (2) faisant partie d'un dispositif de soutien et/ou de support du dispositif (1) pour des parties du corps de l'être humain (6).
